# EUROPEAN PATENT APPLICATION

(11) **EP 4 458 476 A1**
(43) Date of publication of application: **06.11.2024**
(21) Application number: 22914581.8
(22) Date of filing: 23.12.2022
(51) Int. Cl.: B04B 7/00

(54) **ONLINE CENTRIFUGAL SYSTEM**

(30) Priority: 29.12.2021 CN 202111634771; 29.12.2021 CN 202123365403 U
(71) Applicant: Autobio Labtec Instruments Co., Ltd., Zhengzhou, Henan 450016 (CN)
(72) Inventor: XU, Can, Zhengzhou, Henan 450016 (CN); ZHAO, Peng, Zhengzhou, Henan 450016 (CN); CUI, Yanjie, Zhengzhou, Henan 450016 (CN); ZHANG, Shuai, Zhengzhou, Henan 450016 (CN); CUI, Qingdong, Zhengzhou, Henan 450016 (CN); WU, Baojun, Zhengzhou, Henan 450016 (CN); ZHU, Zhiguang, Zhengzhou, Henan 450016 (CN); HOU, Jianping, Zhengzhou, Henan 450016 (CN); WANG, Chao, Zhengzhou, Henan 450016 (CN); LIU, Cong, Zhengzhou, Henan 450016 (CN)
(74) Representative: V.O.
(86) International application number: PCT/CN2022/141644
(87) International publication number: WO 2023/125327

(57) **Abstract**

An online centrifugal system, comprising a conveying device (1), a balancing and weighing mechanism (2), a manipulator (3), and a centrifuge (4). The conveying device (1), the balancing and weighing mechanism (2), and the centrifuge (4) are sequentially arranged. The conveying device (1) is used to convey a sample support (9) for holding blood collection tubes, the balancing and weighing mechanism (2) is used to weigh an adapter (5), and the centrifuge (4) is used to perform a centrifugal operation on blood collection tubes (11). The manipulator (3) is used to grab the blood collection tubes (11) and the adapter (5) respectively, so as to convey the blood collection tubes (11) between the adapter (5) and the conveying device (1), and to convey the adapter (5) between the centrifuge (4) and the balancing and weighing mechanism (2). An inlet and outlet window (401) is provided on the centrifuge (4), and the adapter (5) enters and exits the centrifuge (4) through the inlet and outlet window (401). The balancing and weighing mechanism (2) is used to balance the adapter (5), thereby meeting balancing needs of the adapter (5).

## Description

This application claims the priority to Chinese patent application No. 202111634771.2, titled "ONLINE CENTRIFUGAL SYSTEM", filed on December 29, 2021 with the China National Intellectual Property Administration and Chinese patent application No. 202123365403.0, titled "ONLINE CENTRIFUGAL SYSTEM ", filed on December 29, 2021 with the China National Intellectual Property Administration, the entire disclosures thereof are incorporated herein by reference.

### FIELD

The present disclosure relates to the technical field of devices for online blood centrifugation in a laboratory automation system, and in particular to an online centrifugation system.

### BACKGROUND

Currently, a blood collection tube is required to be placed in an adapter, and the adapter is placed in a centrifuge for blood centrifugation. However, it is hard to guarantee that the adapter meets balance requirements.

Therefore, a technical problem to be solved by those skilled in the art presently is to meet balancing requirements of the adapter.

### SUMMARY

In view of this, an object of the present disclosure is to provide an online centrifugation system capable of meeting balancing requirements of an adapter.

To achieve the above object, the following technical solutions are provided according to the present disclosure.

An online centrifugation system includes a conveying device, a balancing and weighing mechanism, a manipulator, and a centrifuge, the conveying device, the balancing and weighing mechanism, and the centrifuge being arranged in sequence; where
the conveying device is configured to convey a sample holder configured for holding a blood collection tube, the balancing and weighing mechanism is configured to weigh an adapter, and the centrifuge is configured to centrifuge the blood collection tube;
the manipulator is configured to grip the blood collection tube and the adapter separately, to convey the blood collection tube between the adapter and the conveying device, and to convey the adapter between the centrifuge and the balancing and weighing mechanism; and
the centrifuge is provided with an inlet and outlet window configured to allow the adapter to enter and exit the centrifuge through the inlet and outlet window

In a specific solution, the online centrifugation system further includes a sample holder buffering device, and the sample holder buffering device is configured to supply a vacant sample holder to the conveying device.

In another specific solution, the sample holder buffering device is arranged to be perpendicular to or parallel with the conveying device.

In another specific solution, the conveying device includes a first conveying device, a second conveying device, a third conveying device, a first diversion mechanism, a second diversion mechanism, and a third diversion mechanism;
the first conveying device is configured to convey the sample holder holding the blood collection tube, and a first outlet and a first inlet are provided in sequence on a sidewall of the first conveying device along a conveying direction of the first conveying device, where the first outlet is connected to and in communication with an entrance of the second conveying device, the first diversion mechanism is arranged at a junction between the first outlet and an exit of the second conveying device, and is configured to control the sample holder holding the blood collection tube which is not centrifuged to enter the second conveying device;
a second inlet is provided on a sidewall of the second conveying device along a conveying direction of the second conveying device, the second inlet is connected to and in communication with an exit of the sample holder buffering device, the second diversion mechanism is arranged at a junction between the second inlet and the exit of the sample holder buffering device, and is configured to control the vacant sample holder to enter the second conveying device; and
an exit of the second conveying device is connected to and in communication with both an entrance of the sample holder buffering device and the first inlet, the third diversion mechanism is arranged at a junction among the exit of the second conveying device, the entrance of the sample holder buffering device, and the first inlet, and is configured to control the vacant sample holder to enter the sample holder buffering device, and control the sample holder holding the blood collection tube after centrifugation to enter the first conveying device.

In another specific solution, the first diversion mechanism, the second diversion mechanism, and the third diversion mechanism have a same structure and each includes a driving part and a diversion part; where
the driving part is configured to drive the diversion part to move, and the diversion part is configured to guide the sample holder for diversion of the sample holder.

In another specific solution, the online centrifugation system further includes a reading mechanism, and the reading mechanism is arranged on a sidewall of the first conveying device for reading centrifugation information of the blood collection tube on the first conveying device; where
in a case that there's no blood collection tube being held on the sample holder, the rotary scanning mechanism unbinds a blood collection tube barcode from the sample holder, and the third diversion mechanism controls to allow communication between the exit of the second conveying device and the entrance of the sample holder buffering device; and
in a case that the sample holder on the second conveying device holds the blood collection tube, the rotary scanning mechanism binds the blood collection tube barcode to the sample holder, and the third diversion mechanism controls to allow communication between the exit of the second conveying device and the first inlet.

In another specific solution, the online centrifugation system further includes a rotary scanning mechanism, and the rotary scanning mechanism is arranged on the sidewall of the second conveying device for scanning information of the blood collection tube on the second conveying device; where
in a case that there's no blood collection tube being held on the sample holder, the third diversion mechanism controls to allow communication between the exit of the second conveying device and the entrance of the sample holder buffering device; and
in a case that the sample holder on the second conveying device holds the blood collection tube, the third diversion mechanism controls to allow communication between the exit of the second conveying device and the first inlet.

In another specific solution, the sample holder buffering device includes a steering mechanism, a guide rail, and multiple synchronous belts provided in parallel, and the guide rail is arranged on each of both sides of the multiple synchronous belts for guiding the sample holder; and
conveying directions of adjacent synchronous belts are opposite, and the steering mechanism is arranged between the adjacent synchronous belts for steering the sample holders between the adjacent synchronous belts.

In another specific solution, the steering mechanism includes a steering plate, a carrying roller, and a driving assembly, where the carrying roller is arranged between the adjacent synchronous belts, and the driving assembly is in transmission connection with the carrying roller for driving the carrying roller to rotate; and
the steering plate is a curved plate provided on both sides of the steering plate, and is configured for guiding the sample holder from one of the adjacent synchronous belts to the other of the adjacent synchronous belt.

In another specific solution, the balancing and weighing mechanism includes a base plate and a weighing scale; where
the base plate is provided with at least one weighing hole for holding the weighing scale, and the weighing scale is configured to weigh the adapter.

In another specific solution, the base plate is provided with a receiving groove matching the adapter in shape, a bottom of the receiving groove is connected to and in communication with a top of the weighing hole, and a top end of the weighing scale extends into the receiving groove.

In another specific solution, the balancing and weighing mechanism further includes a balancing frame, and the balancing frame is mounted on the base plate for holding a balancing tube.

In another specific solution, the manipulator includes a support, a tube gripper, and an adapter gripper; where
the tube gripper and the adapter gripper are both slidably mounted on the support and are slidable along an X direction, a Y direction, and a Z direction respectively;
the tube gripper is configured to grip the blood collection tube or the balancing tube; and
the adapter gripper is configured to grip the adapter.

In another specific solution, the support includes an X-axis bracket, a Y-axis bracket, and a Z-axis bracket; where
the Y-axis bracket is slidably mounted on the X-axis bracket and is capable of sliding along the X direction, the Z-axis bracket is slidably mounted on the Y-axis bracket and is capable of sliding along the Y direction, and the tube gripper and the adapter gripper are slidably mounted on two sides of the Z-axis bracket and are capable of sliding along the Z direction.

In another specific solution, the online centrifugation system further includes a main control system, an interface control system, and an electronic control system; where
the main control system is communicatively connected to the interface control system, the electronic control system, and the centrifuge, respectively; and
the electronic control system is configured to control operation of the conveying device, balancing and weighing of the balancing and weighing mechanism, action of the manipulator, operation of the sample holder buffering device, action of the reading mechanism, and action of the rotary scanning mechanism.

The various solutions according to the present disclosure can be combined arbitrarily as required, and the solutions obtained after combination are also within the scope of the present disclosure and are a part of the specific solutions of the present disclosure.

Without being limited by any theory, it can be seen from the above disclosure that as the online centrifugation system being used, the sample holder holding the blood collection tube enters the conveying device from the entrance of the conveying device, and the conveying device conveys the sample holder; as the sample holder reaches a position where the manipulator is able to grip, the manipulator grips the blood collection tube on the sample holder and places the blood collection tube on the adapter of the balancing and weighing mechanism for balancing; after the adapter is balanced, the manipulator grips the adapter into the centrifuge for centrifugation; after centrifugation is completed, the manipulator takes the adapter out from the centrifuge and places the adapter on the balancing and weighing mechanism; next, the centrifuged blood collection tube on the adaptor is placed on the vacant sample holder on the conveying device to be conveyed to the next working position. According to the present disclosure, the balancing requirement of the adapter is satisfied by balancing the adapter with the balancing and weighing mechanism.

### BRIEF DESCRIPTION OF THE DRAWINGS

For more clearly illustrating embodiments of the present disclosure or technical solutions in the conventional technology, the drawings referred to for describing the embodiments or the conventional technology will be briefly described hereinafter. Apparently, the drawings in the following description are only some embodiments of the present disclosure, and for those skilled in the art, other drawings may be obtained based on these drawings without any creative efforts.
FIG 1 is a schematic top view showing the structure of an online centrifugation system according to the present disclosure;
FIG 2 is a partial schematic view showing the structure of a sample holder buffering device according to the present disclosure;
FIG 3 is a partial schematic view showing the structure of a steering mechanism according to the present disclosure;
FIG 4 is a three-dimensional view showing the structure of a balancing and weighing mechanism according to the present disclosure;
FIG 5 is a three-dimensional view showing the structure of a manipulator according to the present disclosure;
FIG 6 is a three-dimensional view showing the structure of a transmission blocking device according to the present disclosure; and
FIG 7 is a three-dimensional view showing the structure of a reading mechanism according to the present disclosure.

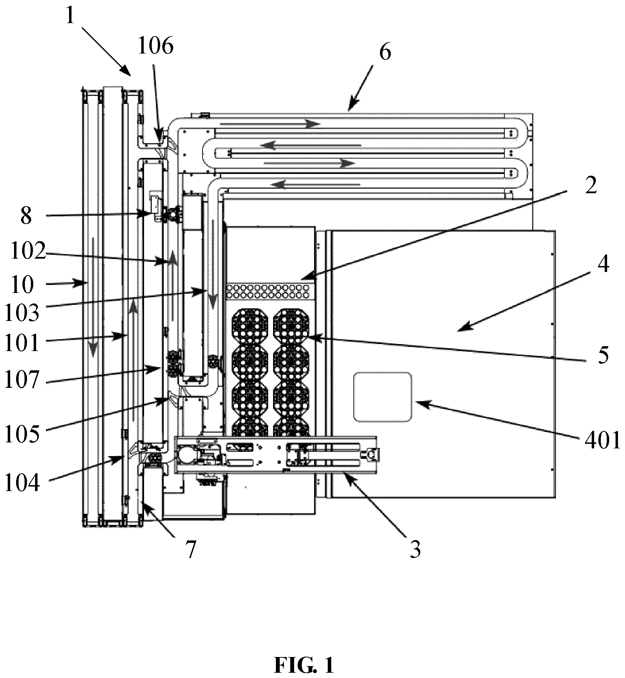

**Reference numerals in FIGS. 1 to 7:**

| | | | |
|---|---|---|---|
| 1 | conveying device, | 101 | first conveying device, |
| 102 | second conveying device, | 103 | third conveying device, |
| 104 | first diversion mechanism, | 105 | second diversion mechanism, |
| 106 | third diversion mechanism, | | |
| 2 | balancing and weighing mechanism, | | |
| 201 | base plate, | 202 | weighing scale, |
| 2011 | receiving groove, | 203 | balancing frame, |
| 3 | manipulator, | 301 | support, |
| 302 | tube gripper, | 303 | adapter gripper, |
| 3011 | X-axis bracket, | 3012 | Y-axis bracket, |
| 3013 | Z-axis bracket, | 4 | centrifuge, |
| 9 | sample holder, | 11 | blood collection tube, |
| 5 | adapter, | 6 | sample holder buffering device, |
| 601 | steering mechanism, | 602 | guide rail, |
| 603 | synchronous belt, | 6011 | steering plate, |
| 6012 | carrying roller, | 6013 | driving assembly, |
| 7 | reading mechanism, | 8 | rotary scanning mechanism, |
| 401 | inlet and outlet window, | | |
| 10 | blood collection tube return conveyor belt, | | |
| 107 | pick-up and drop-off position, | 12 | transmission blocking device, |
| 1201 | gear, | 1202 | first rack, |
| 1203 | second rack, | 1204 | first blocking rod, |
| 1205 | second blocking rod, | 1206 | first sensor, |
| 1207 | second sensor, | 701 | rotating device, |
| 702 | camera, | 703 | blocking member, |
| 704 | light source, | 705 | plane mirror, |
| 7011 | driven wheel, | 7012 | driving wheel, |
| 13 | balancing tube. | | |

### DETAILED DESCRIPTION OF THE EMBODIMENTS

Technical solutions of the embodiments of the present disclosure are clearly and completely described hereinafter in conjunction with the drawings of the embodiments of the present disclosure. Apparently, the embodiments described in the following are only some embodiments of the present disclosure, rather than all embodiments. Any other embodiments obtained by those skilled in the art based on the embodiments in the present disclosure without any creative work fall in the scope of protection of the present disclosure.

In description of the present disclosure, it should be noted that the orientation or positional relationships indicated by terms such as "upper", "lower", "top", "bottom" and the like are based on the orientation or positional relationships shown in the drawings, and are merely for the convenience of describing the present disclosure and simplifying the description, rather than indicating or implying that a device or element referred to must have a particular, or be configured and operated in a particular orientation, which therefore should not be construed as a limitation to the scope of the present application. In addition, the terms "first", "second" and the like are merely for description, and should not be construed as indicating or implying relative importance.

As shown in FIG 1, an online centrifugation system for online centrifugation of a blood collection tube 11 is provided according to the present disclosure.

Specifically, the online centrifugation system includes a conveying device 1, a balancing and weighing mechanism 2, a manipulator 3, and a centrifuge 4.

The conveying device 1, the balancing and weighing mechanism 2, and the centrifuge 4 are arranged in sequence. In order to reduce space occupied by the online centrifugation system, the conveying device 1, the balancing and weighing mechanism 2, and the centrifuge 4 are arranged immediately adjacent to each other, so as to avoid a problem of large space occupation caused by large intervals therebetween.

The conveying device 1 is configured to convey a sample holder 9 for holding the blood collection tube 11. Specifically, the conveying device 1 may be any device capable of conveying the sample holder 9, and specific structure is not limited.

The balancing and weighing mechanism 2 is configured to weigh an adapter 5, and balancing of the adapter 5 is achieved by placing the blood collection tube 11 on the adapter 5 and weighing.

The centrifuge 4 is configured to centrifuge the blood collection tube 11. Specifically, the centrifuge 4 is provided with an inlet and outlet window 401, and the adapter 5 enters and exits the centrifuge 4 through the inlet and outlet window 401.

The manipulator 3 is configured to grip the blood collection tube 11 and the adapter 5, separately, to convey the blood collection tube 11 between the adapter 5 and the conveying device 1, and to convey the adapter 5 between the centrifuge 4 and the balancing and weighing mechanism 2.

It should be noted that the conveying device 1, the balancing and weighing mechanism 2, the manipulator 3, and the centrifuge 4 may share one frame or may have respective frames, which may be configured according to specific requirements.

When the online centrifugation system is in operation, a sample holder holding the blood collection tube 11 enters the conveying device 1 from an entrance of the conveying device 1, and the conveying device 1 conveys the sample holder 9. When the sample holder 9 reaches a position where the manipulator 3 is able to grip, the manipulator 3 grips the blood collection tube 11 on the sample holder 9 and puts it on the adapter 5 of the balancing and weighing mechanism 2. If balancing is needed, the manipulator 3 grips a balancing tube 13 on a balancing frame 203 and puts it to a corresponding position of the adapter 5 for balancing. After the adapter 5 is balanced, the manipulator 3 grips the adapter 5 and places it in the centrifuge 4 for centrifugation. After centrifugation is completed, the manipulator 3 takes the adapter 5 out of the centrifuge 4 and places the adapter 5 on the balancing and weighing mechanism 2. After that, the centrifuged blood collection tube 11 on the adapter 5 is placed on a vacant sample holder 9 on the conveying device 1, and the centrifuged blood collection tube 11 is conveyed to a next working position. The present disclosure meets balancing need of the adapter 5 by balancing the adapter 5 with the balancing and weighing mechanism 2.

In some embodiments, the online centrifugation system further includes a sample holder buffering device 6. The sample holder buffering device 6 is perpendicular to or parallel with the conveying device 1.

The sample holder buffering device 6 is configured to supply a vacant sample holder 9 to the conveying device 1, so that the centrifuged blood collection tube 11 taken from the adapter 5 by the manipulator 3 can be timely placed on the vacant sample holder 9.

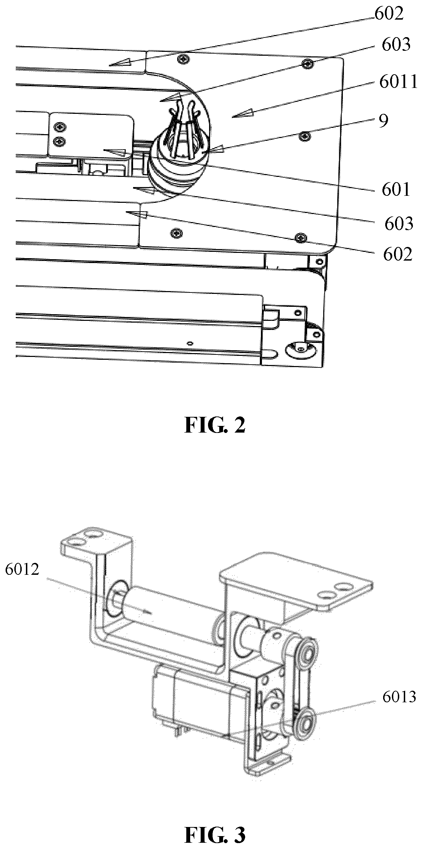

Further, as shown in FIG 2, the present disclosure specifically discloses that the sample holder buffering device 6 includes a steering mechanism 601, a guide rail 602, and multiple synchronous belts 603 provided in parallel. The guide rail 602 is arranged on each of both sides of the synchronous belts 603 for guiding the sample holder 9. The synchronous belts 603 are started to move the sample holder 9 along the guide rails 602, to convey the blood collection tube 11.

It should be noted that the synchronous belt 603 may be replaced by a chain on which a support plate is fixedly laid, where the sample holder 9 is driven by the support plate to move.

Conveying directions of adjacent synchronous belts 603 are opposite, and the steering mechanism 601 is provided between the adjacent synchronous belts 603, to change the conveying direction of the sample holder 9 between the adjacent synchronous belts 603. The multiple synchronous belts 603 make the structure of the whole sample holder buffering device 6 compact.

Further, as shown in FIG 3, the present disclosure discloses that the steering mechanism 601 includes a steering plate 6011, a carrying roller 6012, and a driving assembly 6013. The carrying roller 6012 is arranged between the adjacent synchronous belts 603. The driving assembly 6013 is in transmission connection with the carrying roller 6012, and is configured for driving the carrying roller 6012 to rotate. The carrying roller 6012 drives the sample holder 9 from one synchronous belt 603 to an adjacent synchronous belt 603.

The steering plate 6011 is a curved plate provided on both sides of the steering plate 6011, for guiding the sample holder 9 from one synchronous belt 603 to an adjacent synchronous belt 603.

It should be noted that the steering mechanism 601 is not limited to the above structure, and the steering mechanism 601 may be a conveying belt provided at ends of the synchronous belts 603 at a same side, to realize conveying from one synchronous belt 603 to an adjacent synchronous belt 603.

In some embodiments, a length of the conveying device 1 is greater than a length of the balancing and weighing mechanism 2 or a length of the centrifuge 4, and the length of the balancing and weighing mechanism 2 and the length of the centrifuge 4 should be equal or substantially equal as far as possible. The sample holder buffering device 6 is arranged at an unfilled corner defined by the conveying device 1, the balancing and weighing mechanism 2, and the centrifuge 4, making the whole online centrifugation system compact.

Further, the present disclosure discloses that the conveying device 1 includes a first conveying device 101, a second conveying device 102, a third conveying device 103, a first diversion mechanism 104, a second diversion mechanism 105, and a third diversion mechanism 106.

The first conveying device 101 is configured to convey the sample holder 9 holding the blood collection tube 11, and a first outlet and a first inlet are provided in the listed sequence on a sidewall of the first conveying device 101 along a conveying direction of the first conveying device 101. The first outlet is connected to and in communication with an entrance of the second conveying device 102. The first diversion mechanism 104 is arranged at a junction between the first outlet and an exit of the second conveying device 102, for controlling the sample holder 9 holding the blood collection tube 11 which is not centrifuged yet to enter the second conveying device 102. Specifically, in the case that the blood collection tube 11 on the sample holder 9 conveyed by the first conveying device 101 is not centrifuged, the first diversion mechanism 104 communicates the first outlet with the entrance of the second conveying device 102, to allow the sample holder 9 holding the blood collection tube 11 to enter the second conveying device 102. In the case that the blood collection tube 11 on the sample holder 9 conveyed by the first conveying device 101 has been centrifuged, the first diversion mechanism 104 blocks communication between the first outlet and the entrance of the second conveying device 102, to allow the sample holder 9 holding the blood collection tube 11 to continue being conveyed along the first conveying device 101 to the next working position. It should be noted, in the case that the first diversion mechanism 104 communicates the first outlet with the entrance of the second conveying device 102, the first outlet is blocked from communication with an exit of the first conveying device 101. In the case that the first outlet is blocked from communication with the entrance of the second conveying device 102, the first outlet is in communication with the exit of the first conveying device 101.

A second inlet is provided on a sidewall of the second conveying device 102 along a conveying direction of the second conveying device 102. The second inlet is connected to and in communication with an exit of the sample holder buffering device. The second diversion mechanism 105 is arranged at a junction between the second inlet and the exit of the sample holder buffering device 6, for controlling the vacant sample holder 9 to enter the second conveying device 102. Specifically, in the case that the uncentrifuged blood collection tube 11 is conveyed to the second conveying device 102 by the first conveying device 101, the second inlet is blocked from communication with the exit of the sample holder buffering device 6, and the second inlet is in communication with the exit of the second conveying device 102. Along the conveying direction of the second conveying device 102, a pick-up and drop-off position 107 is provided between the second inlet and the exit of the second conveying device 102, and the pick-up and drop-off position 107 is a position where the manipulator 3 is able to pick up or drop off the blood collection tube 11. In the case that the vacant sample holder 9 on the sample holder buffering device 6 enters the second conveying device 102, an entrance of the sample holder buffering device 6 is in communication with the second inlet, and the second inlet is blocked from communication with the exit of the second conveying device 102.

The exit of the second conveying device 102 is connected to and in communication with both the entrance of the sample holder buffering device 6 and the first inlet. The third diversion mechanism 106 is arranged at a junction among the exit of the second conveying device 102, the entrance of the sample holder buffering device 6, and the first inlet, for controlling the vacant sample holder 9 to enter the sample holder buffering device 6 and controlling the sample holder 9 holding the blood collection tube 11 after centrifugation to enter the first conveying device 101. Specifically, in the case that the sample holder 9 conveyed by the second conveying device 102 is a vacant sample holder 9 that does not hold the blood collection tube 11, the exit of the second conveying device 102 is in communication with the entrance of the sample holder buffering device 6, the exit of the second conveying device 102 is blocked from communication with the first inlet, and the vacant sample holder 9 enters the sample holder buffering device 6. In the case that the sample holder 9 conveyed by the second conveying device 102 holds the centrifuged blood collection tube 11, the exit of the second conveying device 102 is in communication with the first inlet, the exit of the second conveying device 102 is blocked from communication with the entrance of the sample holder buffering device 6, and the sample holder 9 enters the first conveying device 101, to move to the next working position.

Specifically, each of the first conveying device 101, the second conveying device 102, and the third conveying device 103 includes a transmission belt and a slide rail. The slide rail is arranged at each of both sides of the transmission belt, for guiding the sample holder 9. When the transmission belt moves, the sample holder 9 is driven to move along the slide rails.

It should be noted that the transmission belt may be replaced by a sprocket chain. The structures of the first conveying device 101, the second conveying device 102, and the third conveying device 103 may be the same, or may also be different.

Further, the present disclosure discloses that the online centrifugation system further includes a blood collection tube return conveyor belt 10. The blood collection tube return conveyor belt 10 is arranged in parallel with the first conveying device 101 and has a conveying direction opposite to that of the first conveying device 101. The blood collection tube return conveyor belt 10 is configured to convey the blood collection tube 11 output from the exit of the first conveying device 101 to the next working positions.

In some embodiments, the first diversion mechanism 104, the second diversion mechanism 105, and the third diversion mechanism 106 have the same structure, and each includes a driving part and a diversion part.

The driving part is configured to drive the diversion part to move and the diversion part guides the sample holder 9, to realize direction changing of the sample holder 9. The sample holder 9 on the first conveying device 101 being moved to the second conveying device 102 is taken as an example, the driving part drives the diversion part to rotate onto the first conveying device 101, so that the diversion part blocks communication between the entrance and the exit of the first conveying device 101, and the first outlet of the first conveying device 101 is in communication with the entrance of the second conveying device 102. The blocking part is arc-shaped and extends onto the second conveying device 102, to allow the sample holder to enter the second conveying device 102 along the blocking part by conveyance of the first conveying device 101.

It should be noted that the driving part and the diversion part may be of other structures.

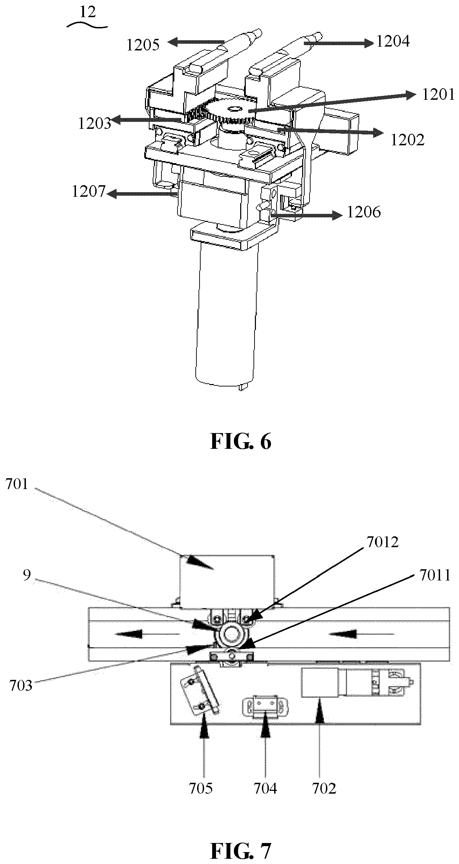

In order to realize equal distance between sample holders 9 on the conveying device 1, the present disclosure discloses that the online centrifugation system further includes a transmission blocking device 12. As shown in FIG 6, the transmission blocking device 12 includes a mounting support, a motor, a gear 1201, a first rack 1202, a second rack 1203, a first blocking rod 1204, and a second blocking rod 1205. The first rack 1202 and the second rack 1203 are slidably mounted on the mounting support, respectively. The gear 1201 is arranged between the first rack 1202 and the second rack 1203, and the gear 1201 meshes with the first rack 1202 and the second rack 1203 respectively for transmission. The first blocking rod 1204 is fixed on the first rack 1202 and the second blocking rod 1205 is fixed on the second rack 1203.

A distance between the first blocking rod 1204 and the second blocking rod 1205 may be configured to accommodate one sample holder 9 or two sample holders 9, etc.

In a moving direction of the sample holder 9, the first blocking rod 1204 is in front of the second blocking rod 1205, and the case where the distance between the first blocking rod 1204 and the second blocking rod 1205 is configured to accommodate one sample holder 9 is taken as an example. During transmission of the sample holder 9, if it is needed to prevent the sample holder 9 from moving forward, the first blocking rod 1204 sticks out and the second blocking rod 1205 retracts, so that all the following sample holders 9 are blocked. If the sample holder 9 is required to keep moving forward in the current direction, the first blocking rod 1204 retracts and the second blocking rod 1205 sticks out to allow the frontmost sample holder to pass and the following sample holders 9 to be blocked by the second blocking rod 1205. With the above process being circulated in sequence, one single sample holder 9 being released for transmission at a time is realized.

To facilitate control, the present disclosure discloses that the online centrifugation system further includes a first sensor 1206 and a second sensor 1207. The first sensor 1206 and the second sensor 1207 are symmetrically mounted on two sides of the mounting support, for detecting the first blocking rod 1204 and the second blocking rod 1205, respectively. The first blocking rod 1204 is detected by the first sensor 1206 when the first blocking rod 1204 is fully retracted without blocking effect, and the second blocking rod 1205 is detected by the second sensor 1207 when the second blocking rod 1205 is fully retracted without blocking effect.

In some embodiments, the online centrifugation system further includes a reading mechanism 7. The reading mechanism 7 is arranged on a sidewall of the first conveying device 101 for reading centrifugation information of the blood collection tube 11 on the first conveying device 101.

In the case it is read that the blood collection tube 11 is a centrifuged blood collection tube 11, the first diversion mechanism 104 control to block communication between the first outlet and the exit of the second conveying device 102, to make the blood collection tube 11 continue being conveyed along the conveying direction of the first conveying device 101.

In the case it is read that the blood collection tube 11 is an uncentrifuged blood collection tube 11, the first diversion mechanism 104 controls to allow communication between the first outlet and the exit of the second conveying device 102, and the blood collection tube 11 enters the second conveying device 102.

The reading mechanism 7 is able to photograph information of the entire circumferential direction of the blood collection tube 11. Specifically, multiple cameras 702 may be provided to photograph information from different angles of the blood collection tube 11, so as to determine whether the blood collection tube 11 is a centrifuged blood collection tube 11. It should be noted that it is also possible to provide only one camera 702, with a rotating device 701 being provided to rotate the sample holder 9, photographing of the blood collection tube 11 from different angles are realized. That is to say, the reading mechanism 7 includes the rotating device 701, the camera 702, and a blocking member 703. The blocking member 703 is mounted on one side of the conveying device 1 in a retractable manner, and is capable of blocking the sample holder 9. As shown in FIG 7, in order to improve photographing quality, the present disclosure discloses that the reading mechanism 7 also includes a light source 704 and a plane mirror 705. The light source 704 illuminates a to-be-observed side of the blood collection tube 11, to provide a bright environment for photographing. The sample holder 9 and the camera 702 are symmetrically disposed with respect to a central axis of the plane mirror 705, that is, the camera 702 is capable of observing the blood collection tube 11 through the plane mirror 705. An AI vision system is adopted to introduce determination of height and diameter of the blood collection tube 11, presence or absence of a tube cap, color of the tube cap, and the blood collection tube 11 being placed in position or not, so as to facilitate automated information processing of automated workflow. By scanning through the plane mirror 705, a problem that a common visual scanning device occupies a large longitudinal space on the rail is effectively solved. The light source 704 supplies complementary light to the blood collection tube 11 directly and the camera 702 scans the blood collection tube 11 indirectly, effectively preventing information scanning of the blood collection tube 11 from being interfered by shadow of the blood collection tube 11.

Further, the present disclosure discloses that the rotating device 701 includes a driven wheel 7011 and a driving wheel 7012. The driven wheel 7011 is provided on one side of the conveying device 1, and the driving wheel 7012 arranged on the other side of the conveying device 1 in a retractable manner. If the sample holder 9 needs to be rotated, the driving wheel 7012 sticks out to press against the sample holder 9, and the other side of the sample holder 9 is pressed against the driven wheel 7011. The driving wheel 7012 rotates under driving of a motor or the like, to drive the sample holder 9 to rotate. Specifically, the number of the driving wheel 7012 is two. In the case that the driving wheels 7012 press against the sample holder 9, the two driving wheels 7012 are located at front and rear ends of the driven wheel 7011 respectively, thus forming a triangle. A device for driving the driving wheel 7012 to stick out or retract may be a gear and rack mechanism, a linear actuator, or other structures.

In some embodiments, the online centrifugation system further includes a rotary scanning mechanism 8. The rotary scanning mechanism 8 is located on a sidewall of the second conveying device 102, for scanning information of the blood collection tube 11 on the sample holder 9 on the second conveying device 102.

In the case that there's no blood collection tube 11 on the sample holder 9 on the second conveying device 102, the rotary scanning mechanism 8 unbinds a blood collection tube 11 barcode from the sample holder 9, and the third diversion mechanism 106 control to allow communication between the exit of the second conveying device 102 and the entrance of the sample holder buffering device. In the case that the sample holder 9 on the second conveying device 102 holds the blood collection tube 11, the rotary scanning mechanism 8 binds the blood collection tube 11 barcode to the sample holder 9, and the third diversion mechanism 106 control to allow communication between the exit of the second conveying device 102 and the first inlet.

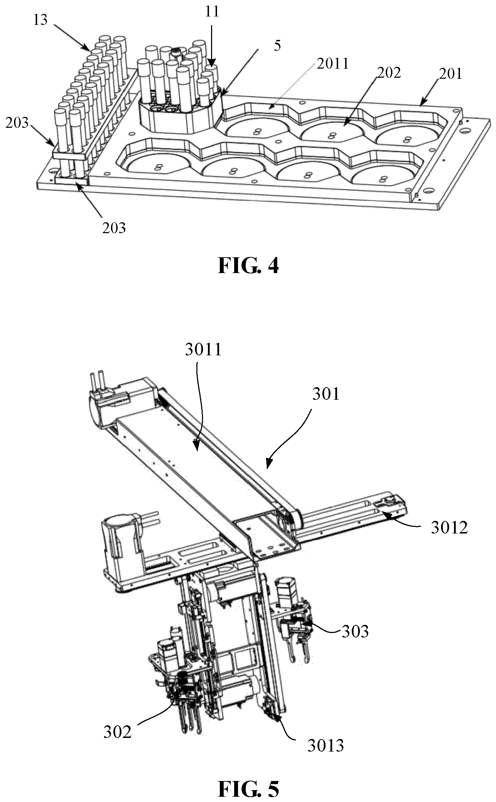

In some embodiments, as shown in FIG 4, the balancing and weighing mechanism 2 includes a base plate 201 and a weighing scale 202. The base plate 201 is provided with at least one weighing hole for holding the weighing scale 202, and the weighing scale 202 is configured to weigh the adapter 5.

Further, the present disclosure discloses that the base plate 201 is provided with a receiving groove 2011 matching the adapter 5 in shape. A bottom of the receiving groove 2011 is connected to and in communication with a top of the weighing hole. A top end of the weighing scale 202 extends into the receiving groove 2011. The receiving groove 2011 is provided to facilitate positioning of the adapter 5.

Further, the present disclosure discloses that the balancing and weighing mechanism 2 further includes a balancing frame 203. The balancing frame 203 is mounted on the base plate 201, for holding a balancing tube 13.

In some embodiments, as shown in FIG 5, the manipulator 3 includes a support 301, a tube gripper 302, and an adapter gripper 303. Both of the tube gripper 302 and the adapter gripper 303 are slidably mounted on the support 301 and are slidable along an X direction, a Y direction, and a Z direction, respectively, to enable movements of the tube gripper 302 and the adapter gripper 303 in spatial location in the X direction, the Y direction, and the Z direction.

The tube gripper 302 is configured to grip the blood collection tube 11 or the balancing tube 13, and the adapter gripper 303 is configured to grip the adapter 5.

Further, the present disclosure discloses that the support 301 includes an X-axis bracket 3011, a Y-axis bracket 3012, and a Z-axis bracket 3013. The Y-axis bracket 3012 is slidably mounted on the X-axis bracket 3011 and is slidable along the X direction. The Z-axis bracket 3013 is slidably mounted on the Y-axis bracket 3012 and is slidable along the Y direction. The tube gripper 302 and the adaptor gripper 303 are slidably mounted on two sides of the Z-axis bracket 3013 respectively and are slidable along the Z direction. It should be noted that movement of the Y-axis bracket 3012 and the Z-axis bracket 3013 may be driven by a motor or the like.

Further, the present disclosure discloses that number of the tube gripper 302 is two, so that two blood collection tubes 11 can be gripped at a time, which improves gripping efficiency of the blood collection tubes 11.

In some embodiments, the online centrifugation system further includes a main control system, an interface control system, and an electronic control system.

The main control system is communicatively connected to the interface control system, the electronic control system, and the centrifuge 4, respectively.

The electronic control system is configured to control operation of the conveying device 1, balancing and weighing of the balancing and weighing mechanism 2, action of the manipulator 3, operation of the sample holder buffering device 6, action of the reading mechanism 7, and action of the rotary scanning mechanism 8.

Specifically, the interface control system displays operation status of each component in real time. With interface operation, control of the conveying device 1, control of the manipulator 3, parameter setting and process control of the centrifuge 4, timing sequence, control of auxiliary routines, display and switching of individual list, control of nodes as well as parameter editing and sending, numbering of local IP ports and node, and so on.

A sample transmission module includes four functions, namely, transmission of sample holder 9, buffering of sample holder 9, check-in and check-out of the sample holder 9, and check-in and check-out of the adapter 5. When the sample holder 9 on the conveying device 1 is detected, RFID information of current sample is read and reported. By parsing received routing instructions, the sample holder 9 that needs to be centrifuged is transmitted to a sample-picking track, and the blood collection tube 11 to be centrifuged is placed on the adapter 5 by the manipulator 3. The sample check-in process is completed, the sample holder 9 is unbound, and the vacant sample holder 9 is transmitted to the buffering area. When check-in of the sample on the adapter 5 is completed, the centrifuge 4 is controlled to be in position, the adapter 5 is checked-in into the centrifuge 4, and the centrifugation process begins. After centrifugation, the adapter 5 is checked-out from the centrifuge 4. If a vacant sample holder 9 is detected in position, the centrifuged sample is placed on the sample holder 9. Then the centrifuged sample holder 9 and the vacant sample holder 9 undergo barcode scanning, RFID binding and reporting, and are transmitted to the conveying device 1 in a routing manner.

It should be noted that the words indicating the orientation in this specification, such as "upper" and "lower", are all referred to directions in FIG 1, which are only for the convenience of description and do not have other specific meanings.

The above embodiments are described in a progressive manner. Each of the embodiments is mainly focused on describing its differences from other embodiments, and references may be made among these embodiments with respect to the same or similar parts.

Based on the above description of the disclosed embodiments, those skilled in the art can implement or use the present disclosure. It is obvious for those skilled in the art to make many modifications to these embodiments. The general principles defined herein may be applied to other embodiments without departing from the spirit or scope of the present disclosure. Therefore, the present disclosure is not limited to the embodiments described herein, but should be defined by the broadest scope consistent with the principle and novel features disclosed herein.

In the description of this specification, description with reference to the terms "one embodiment", "example", "specific example", etc. means that specific features, structures, materials or characteristics described in connection with this embodiment or example are included in at least one embodiment or example of the present disclosure. In this specification, the schematic expressions of the above terms do not necessarily refer to the same embodiments or examples. Furthermore, the specific features, structures, materials or characteristics described may be combined in any one or more embodiments or examples in a suitable manner.

## Claims

1. An online centrifugation system, comprising a conveying device, a balancing and weighing mechanism, a manipulator, and a centrifuge; wherein
the conveying device, the balancing and weighing mechanism, and the centrifuge are arranged in sequence; and wherein
the conveying device is configured to convey a sample holder configured for holding a blood collection tube, the balancing and weighing mechanism is configured to weigh an adapter, and the centrifuge is configured to centrifuge the blood collection tube;
the manipulator is configured to grip the blood collection tube and the adapter separately, to convey the blood collection tube between the adapter and the conveying device, and convey the adapter between the centrifuge and the balancing and weighing mechanism; and
the centrifuge is provided with an inlet and outlet window configured to allow the adapter to enter and exit the centrifuge.

2. The online centrifugation system according to claim 1, wherein the online centrifugation system further comprises a sample holder buffering device, and the sample holder buffering device is configured to supply a vacant sample holder to the conveying device.

3. The online centrifugation system according to claim 2, wherein the sample holder buffering device is arranged to be perpendicular to or parallel with the conveying device.

4. The online centrifugation system according to claim 2, wherein the conveying device comprises a first conveying device, a second conveying device, a third conveying device, a first diversion mechanism, a second diversion mechanism, and a third diversion mechanism; and wherein
the first conveying device is configured to convey the sample holder holding the blood collection tube, and a first outlet and a first inlet are provided in sequence on a sidewall of the first conveying device along a conveying direction of the first conveying device, wherein the first outlet is connected to and in communication with an entrance of the second conveying device, the first diversion mechanism is arranged at a junction between the first outlet and an exit of the second conveying device, and is configured to control the sample holder holding the blood collection tube which is not centrifuged to enter the second conveying device;
a second inlet is provided on a sidewall of the second conveying device along a conveying direction of the second conveying device, the second inlet is connected to and in communication with an exit of the sample holder buffering device, the second diversion mechanism is arranged at a junction between the second inlet and the exit of the sample holder buffering device, and is configured to control the vacant sample holder to enter the second conveying device; and
an exit of the second conveying device is connected to and in communication with both an entrance of the sample holder buffering device and the first inlet, the third diversion mechanism is arranged at a junction among the exit of the second conveying device, the entrance of the sample holder buffering device, and the first inlet, and is configured to control the vacant sample holder to enter the sample holder buffering device, and control the sample holder holding the blood collection tube after centrifugation to enter the first conveying device.

5. The online centrifugation system according to claim 4, wherein the first diversion mechanism, the second diversion mechanism, and the third diversion mechanism have a same structure and each comprises a driving part and a diversion part; and wherein
the driving part is configured to drive the diversion part to move, and the diversion part is configured to guide the sample holder for diversion of the sample holder.

6. The online centrifugation system according to claim 4, further comprising a reading mechanism, wherein the reading mechanism is arranged on a sidewall of the first conveying device for reading centrifugation information of the blood collection tube on the first conveying device; and wherein
in a case it is read that the blood collection tube is centrifuged, the first diversion mechanism is configured to control the first outlet and the exit of the second conveying device to be shut off, to allow the blood collection tube to continue being conveyed along the conveying direction of the first conveying device; and
in a case it is read that the blood collection tube is not centrifuged, the first diversion mechanism is configured to control to allow communication between the first outlet and the exit of the second conveying device, to allow the blood collection tube to enter the second conveying device.

7. The online centrifugation system according to claim 4, further comprising a rotary scanning mechanism, wherein the rotary scanning mechanism is arranged on the sidewall of the second conveying device for scanning information of the blood collection tube on the second conveying device; and wherein
in a case that there's no blood collection tube being held on the sample holder on the second conveying device, the rotary scanning mechanism is configured to unbind a blood collection tube barcode from the sample holder, and the third diversion mechanism is configured to control to allow communication between the exit of the second conveying device and the entrance of the sample holder buffering device; and
in a case that the sample holder on the second conveying device holds the blood collection tube, the rotary scanning mechanism is configured to bind the blood collection tube barcode to the sample holder, and the third diversion mechanism is configured to control to allow communication between the exit of the second conveying device and the first inlet.

8. The online centrifugation system according to claim 2, wherein the sample holder buffering device comprises a steering mechanism, a guide rail, and a plurality of synchronous belts provided in parallel; and wherein
the guide rail is arranged on each of both sides of the plurality of synchronous belts for guiding the sample holder; and
conveying directions of adjacent synchronous belts of the plurality of synchronous belts are opposite, and the steering mechanism is arranged between the adjacent synchronous belts for steering the sample holder between the adjacent synchronous belts.

9. The online centrifugation system according to claim 8, wherein the steering mechanism comprises a steering plate, a carrying roller, and a driving assembly; and wherein
the carrying roller is arranged between the adjacent synchronous belts, and the driving assembly is in transmission connection with the carrying roller for driving the carrying roller to rotate; and
the steering plate is a curved plate arranged on both sides of the steering plate, and is configured for guiding the sample holder from one of the adjacent synchronous belts to the other of the adjacent synchronous belts.

10. The online centrifugation system according to claim 1, wherein the balancing and weighing mechanism comprises a base plate and a weighing scale; and wherein
the base plate is provided with at least one weighing hole for holding the weighing scale, and the weighing scale is configured to weigh the adapter.

11. The online centrifugation system according to claim 10, wherein the base plate is provided with a receiving groove matching the adapter in shape, a bottom of the receiving groove is connected to and in communication with a top of the weighing hole, and a top end of the weighing scale extends into the receiving groove.

12. The online centrifugation system according to claim 10, wherein the balancing and weighing mechanism further comprises a balancing frame, and the balancing frame is mounted on the base plate for holding a balancing tube.

13. The online centrifugation system according to claim 12, wherein the manipulator comprises a support, a tube gripper, and an adapter gripper; and wherein
the tube gripper and the adapter gripper are both slidably mounted on the support and are slidable along an X direction, a Y direction, and a Z direction respectively;
the tube gripper is configured to grip the blood collection tube or the balancing tube; and
the adapter gripper is configured to grip the adapter.

14. The online centrifugation system according to claim 13, wherein the support comprises an X-axis bracket, a Y-axis bracket, and a Z-axis bracket; and wherein
the Y-axis bracket is slidably mounted on the X-axis bracket and is slidable along the X direction, the Z-axis bracket is slidably mounted on the Y-axis bracket and is slidable along the Y direction, and the tube gripper and the adapter gripper are slidably mounted on two sides of the Z-axis bracket respectively and are slidable along the Z direction.

15. The online centrifugation system according to any one of claims 1 to 14, further comprising a main control system, an interface control system, and an electronic control system; and wherein
the main control system is communicatively connected to the interface control system, the electronic control system, and the centrifuge, respectively; and
the electronic control system is configured to control operation of the conveying device, balancing and weighing of the balancing and weighing mechanism, action of the manipulator, operation of the sample holder buffering device, action of the reading mechanism, and action of the rotary scanning mechanism.
